# EUROPEAN PATENT SPECIFICATION

(11) **EP 3 445 224 B1**
(45) Date of publication and mention of the grant of the patent: **25.09.2024**
(21) Application number: 16750393.7
(22) Date of filing: 19.07.2016
(51) Int. Cl.: A61B 5/00, B60K 28/06, A61B 5/024, A61B 5/11, B60K 28/04, B60T 7/14, B60L 3/02

(54) **DEAD MAN'S CONTROL SYSTEM AND METHOD FOR A VEHICLE**
TOTMANNSTEUERUNGSSYSTEM UND -VERFAHREN FÜR EIN FAHRZEUG
SYSTÈME DE BLOCAGE SÉCURITÉ ET PROCÉDÉ POUR UN VÉHICULE

(30) Priority: 20.04.2016 EP 16380017
(43) Date of publication of application: 27.02.2019
(73) Proprietor: Siemens Rail Automation S.A.U., 28760 Tres Cantos (Madrid) (ES)
(72) Inventor: PASTOR PEREZ, Victor Manuel, 28770 Colmenar Viejo (ES)
(74) Representative: Siemens Patent Attorneys
(86) International application number: PCT/EP2016/067157
(87) International publication number: WO 2017/182104

(56) References cited:
- US-A1- 2014 276 090
- US-A1- 2015 088 397
- US-A1- 2015 314 681
- US-A1- 2015 328 985

## Description

The present invention concerns a dead man's detection system and method for a vehicle according to the independent claims.

The present invention is directed to dead man's control systems, also called sometimes "Driver's Safety Devices", that are automatically operated in case an operator of a vehicle incorporating the dead man's control system becomes incapacitated. Dead man's control systems usually aim to stop the vehicle operated by said operator in order to prevent any harmful situation as soon as a critical situation occurs regarding the capacity of the operator to drive said vehicle.

Dead man's control systems may equip different kind of vehicles. Indeed, vehicle according to the present invention might be guided vehicles, i.e. a device for carrying or transporting substances, objects or individuals that is guided by at least one guiding means, such as a rail, and which refer more particularly to public transport means such as buses, trolleybuses, streetcars, subways, trains or train units, etc., as well as load transporting means such as, for example, overhead traveling cranes or mining transportation means, or aircrafts, or any moving vehicle that comprises an operator in charge of driving said vehicle. The dead man's control system is in charge of making sure the operator driving said vehicle is fully conscious.

Examples of dead man's control systems are for instance:
- a pedal system, wherein the vehicle operator has to press and release the pedal within a period of time. If this action, i.e. pressing and releasing, is not performed within said period of time, the vehicle automatically applies emergency brakes or enter into a safe state;
- a handle system, wherein the vehicle operator has to maintain a pressure on said handle. If the pressure is lost or released, then the vehicle automatically brakes or enter into said safe state;
- a button system, wherein the vehicle operator has to push said button within a period of time. If the vehicle operator does not push said button within said period of time, the operator might be alerted by a buzz. If after said buzz the button is still not pushed, the system automatically applies emergency brakes or place the vehicle in the safe state;
- a touch sensor system, wherein a speed controller is for instance fitted with a capacitive touch sensor. If the vehicle operator's hand is not in contact with the touch sensor, then the vehicle automatically brakes or enters a safe state.

The prior art dead man's control systems all require an active action or reaction from the vehicle operator. This might lead to some failures of the system, for instance in the case the vehicle operator is unconscious, but still touching the above-mentioned capacitive touch sensor. For example, US2015/088397 relates to a system for monitoring, recording and/ or analyzing vigilance, alertness or wakefulness and/or a stressed state of an operator of equipment or machinery in a variety of situations including situations wherein the degree of vigilance of the operator has implications for the safety or well being of the operator or other persons.

An objective of the present invention is to provide a safer dead man's control system and method compared to the known prior art systems and methods.

The aforementioned objective is achieved by a dead man's control system and a method according to the independent claims. Further embodiments and other advantages of the present invention are proposed in the dependent claims.

The invention proposes in particular a dead man's control system, configured for being installed on-board a vehicle designed for being operated by an operator, for instance from an operator cabin, the dead man's control system being configured for checking a capacity of the vehicle operator to operate said vehicle and comprising:
- a pulsation rate measurement device configured for measuring heartbeat of the operator and outputting in real time pulsation rate data;
- a movement detection system for detecting a movement, i.e. a change of position, of at least one part of the body of the operator and outputting in real time movement detection data;
- a processing unit for analyzing in real time the pulsation rate data provided in real time by the pulsation rate measurement device and the movement detection data provided in real time by the movement detection system, wherein the processing unit is configured for automatically triggering a safety system of the vehicle in function of its analysis of the pulsation rate data and the movement detection data.

The processing unit is in particular able to determine a presence or absence of at least one of the operators in said operator cabin from its analysis of said pulsation rate data and/or movement detection data and for automatically triggering said safety system in case of an absence of at least one of said operators or a driving incapacity of each operator remaining inside the cabin.

The present invention concerns also a dead man's control method capable of detecting in real time a driving incapacity of an operator of a vehicle during operation or driving of said vehicle by said operator, the method comprising:
- measuring in real time the heartbeat of the operator, for instance using a non-invasive method for heart rate monitoring, and outputting in real time pulsation rate data regarding said heartbeat measurement;
- detecting in real time a movement, i.e. a change of position, of at least one part of the body of the operator and outputting in real time movement detection data for the movement of said at least one part of the body;
- analyzing in real time the pulsation rate data and the movement detection data;
- automatically triggering a safety system of the vehicle in function of the analysis of the pulsation rate data and the movement detection data.

Further aspects and advantages of the present invention will be better understood through the following drawings, wherein like numerals are used for like and corresponding parts:
- Figure 1: schematic representation of a preferred embodiment of a dead man's control system according to the invention mounted on board a guided vehicle.
- Figure 2: detailed view of a cabin of a vehicle equipped with a preferred embodiment of the dead man's control system according to the invention.

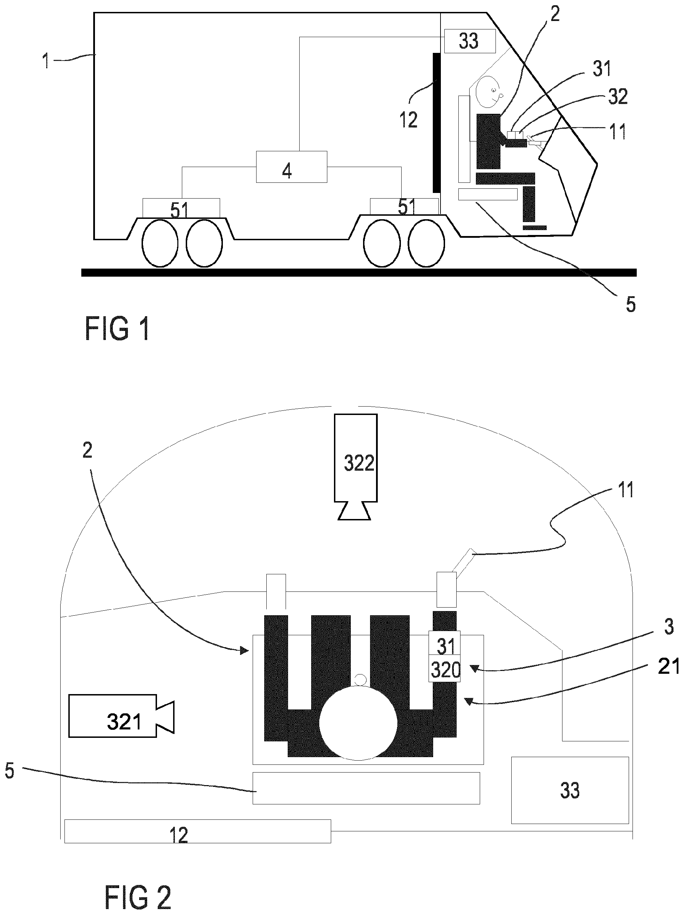

Figure 1 shows a vehicle 1 equipped with a preferred embodiment of a dead man's control system according to the invention, and whose task is to control a capacity of a vehicle operator 2 or driver to operate or drive said vehicle 1.

The dead man's control system might be installed on board the vehicle 1, e.g. in an operator cabin that is closed by a door 12 from the rest of the vehicle, the locking of said door being for instance controlled by a locking system, said door 12 physically separating for instance the operator 2 from passengers of the vehicle 1. The dead man's control system might also equip a remote control cabin that is configured for remotely controlling the vehicle 1.

The dead man's control system according to the invention typically comprises a pulsation rate measurement device 31 and a movement detection device 32, wherein the movement detection device 32 may comprise an accelerometer 320 and/or a camera system that may comprise a first camera 321 and a second camera 322. The cameras 321, 322 of the camera system are preferentially installed inside the operator cabin in order to acquire images of the operator 2, said camera system being configured for enabling a check of the capacity of the operator 2 to drive the vehicle 1 from positions or change of positions of the operator on his seat. For instance, the first camera 321 is positioned in the cabin for pointing to the operator's side and the second camera 321 is positioned in the cabin for pointing to the operator's face. Preferentially, the dead man's control system comprises a portable device 3 that comprises itself the accelerometer 320 and the pulsation rate device 31. Said portable device 3 might be worn by the operator 2, e.g. being a wristband wearable on his wrist. Preferentially said portable device 3 is configured for being worn on a body part of the operator which is moving during the driving of the vehicle and which motion controls the driving or operation of the vehicle. Preferentially, the portable device 3 is able to wirelessly communicate with the processing unit 33 in order to provide to said processing unit the pulsation rate data and a first set of movement detection data originating from the accelerometer 320.

The movement detection system 32 and the pulsation rate device 31 are configured for outputting in real time respectively movement detection data and pulsation rate data which are usable for determining respectively a change of position of at least one body part of the operator and the heartbeat of said operator by means of a processing unit 33. Typically, the pulsation rate device is configured for measuring heartbeat in said body part, e.g. from blood motion, using well known non-invasive techniques, for instance by using infrared light. For instance, when incorporated to the portable device 3 being a wristband, the pulsation rate device is able to measure heart pulsations at said wrist of the operator. Then, the processing unit 33 according to the invention is configured for analyzing in real time the pulsation rate data and the movement detection data, in order to automatically trigger a safety system 4 of the vehicle 2 if preconfigured conditions regarding the movement detection data and the pulsation rate data are not fulfilled. In other words, the processing unit 33 is able to analyze the pulsation rate data and the movement detection data and to determine, in function of said analysis, if said safety system 4 has to be triggered or not. In particular, the triggering of the safety system 4 is configured for carrying out at least one of the following actions:
i) deactivating the locking system of the door 12 of the operator cabin, notably if the processing unit 33 determines that at least one operator is missing in said cabin. In particular, the processing unit 33 according to the invention is able to determine a presence or absence of at least one operator in the cabin from the pulsation rate data and/or the movement detection data. For instance, the pulsation rate device 31 is in particular configured for being able to emit or communicate the pulsation rate data to the processing unit only if the pulsation rate device 31 is located in the operator cabin, for instance at some positions allowing its powering, or, and in particular, when the operator 2 is sitting at a specific location in the cabin that allows him to drive the vehicle. For instance, gripping a handle 11 of the vehicle by the operator may activate a switch of the movement detection system and/or of the pulsation rate device that triggers the transmission of pulsation rate data and movement detection data to the processing unit. For instance, the pulsation rate data and the first set of movement detection data might be wirelessly communicated to the processing unit, wherein RFID techniques are used for enabling said communication only if the pulsation rate device and the movement detection device are located in the operator cabin. Additionally, or optionally, the processing unit 33 according to the invention is in particular able to determine a presence or an absence of at least one operator in the cabin by analyzing the pulsation rate data, wherein the pulsation rate data are compared to a pulsation rate pattern stored in a database of the processing unit 33 for each operators and an absence of the operator is determined by the processing unit 33 if the pulsation rate data does not match with the operator pulsation rate pattern. Preferentially, the processing unit 33 is configured for determining an absence of the operator if the pulsation rate data is not being received and/or the first set of movement detection data is not being received and/or the second set of movement detection data is not correct.
ii) activating an emergency brake system 51 of the vehicle and or activating an alert for the operator as it will be explained in more details later. Preferentially, the processing unit 3 comprises an interface with a braking system of the vehicle;
iii) putting the vehicle in a fail-safe state, for instance by activating an automatic pilot, in particular if an operator or all operators left the cabin. Preferentially, the processing unit 33 comprises an interface with an automatic pilot switch;
iv) automatically sending an alarm message to an emergency service.

According to the present invention, the movement detection system 32 is able to detect at least a change of position of said body part of the operator 2, in particular by means of said accelerometer 320. Additionally and optionally, the first camera 321 is able to provide images enabling analysis by the processing unit 33 of the operator's position in its seat 5 as well as body motion, and the second camera 322 is configured for providing images suitable for an analysis by the processing unit 33 of the operator's head alignment and face gestures. In particular, the movement detection data comprise said first set of data and a second set of data, wherein the first set of data is provided by said accelerometer and will be called "accelerometer" data, while the second set of data are data provided by the camera system, for instance by the first camera 321 and the second camera 322, and will be called "video data". In other words, the second set of data that are video data encoding images acquired in real time by the video system and provided in real time to the processing unit 33. Preferentially, the video data might be used by the processing unit 33 for the determination of an absence or presence of the operator in the cabin. For this purpose, the processing unit might use algorithms for the detection of person.

The conditions verified by the processing unit 33 and required for triggering the safety system 4 by means of said processing unit will be now described in more details. According to the present invention, the triggering of the safety system 4 by the processing unit 33 is preferentially configured for activating the emergency brake system 51 when at least the following situations occur:
(a) if the processing unit determines notably after a predetermined period of time that there are
   - no or abnormal pulsations from the pulsation rate data, for instance and in particular, if it does not receive any pulsation rate data, and
   - no detectable movement from the first set of data, for instance and in particular, if it does not receive data belonging to said first set of data or the body part is immobile, and
   - an incorrect body position from the second set of data, for instance and in particular, if it does not receive data belonging to said second set of data,
   then the triggering of said safety system 4 by the processing unit is configured for activating the emergency brake system (indeed, the cause of the above-mentioned situation might be the death of the operator or the operator having left the cabin); or
(b) if the processing unit determines notably after a predetermined period of time that there are
   - no or abnormal pulsations from the pulsation rate data, for instance and in particular, if it does not receive any pulsation rate data, and
   - no detectable movement from the first set of data, for instance and in particular, because it does not receive data belonging to said first set of data or because the body part is immobile, and
   - a correct body position from the second set of data,
   then the triggering of said safety system 4 by the processing unit is configured for activating the emergency brake system (indeed, the cause of such a situation might be a problem with the portable device 3 or a death of the operator while the body remains in a correct position); or
(c) if the processing unit determines notably after a predetermined period of time that there are
   - no or abnormal pulsations from the pulsation rate data, for instance and in particular, if it does not receive any pulsation rate data, and
   - a movement from the first set of data, for instance, the body part on which the portable device 3 is worn is moving, and
   - an incorrect body position from the second set of data, for instance and in particular, if it does not receive data belonging to said second set of data,
   then the triggering of said safety system 4 by the processing unit is configured for activating the emergency brake system (indeed, the cause of the above-mentioned situation might be that the operator is suffering, for instance from an epileptic seizure, or for other reasons, the accelerometer is detecting movement); or
(d) if the processing unit determines notably after a predetermined period of time that there are
   - normal pulsations from the pulsation rate data,
   - no detectable movement from the first set of data, for instance and in particular, because it does not receive data belonging to said first set of data or because the body part is immobile, and
   - an incorrect body position from the second set of data, for instance and in particular, if it does not receive data belonging to said second set of data,
   then the triggering of said safety system 4 by the processing unit is configured for activating an emergency brake system (indeed, the cause of the above-mentioned situation might be that the driver is unconscious).

By "correct", or "incorrect" position, it has to be understood that the processing unit 33 is able to determine from the second set of data if the position of the operator in his seat, his head alignment with his body and a face gesture correspond to a normal situation wherein the operator is conscious and capable of driving the vehicle or to an abnormal situation wherein the operator is not anymore capable of driving the vehicle. Learning algorithms might be used for the classification of the second set of data in a first class that groups the so-called "normal situations" or in a second class that groups the so-called "abnormal situations". Such learning algorithm are known by the skilled person and not described here. By normal or abnormal pulsation, it has to be understood that the processing unit is configured for determining if the heartbeat of the operator belongs to the so called "normal situations" or "abnormal situations". For this purpose, the processing unit might comprise in a database pulsation rate threshold defined for each operator of the vehicle, as well as historical measurements of pulsation rate for each of the operators, for instance specific pulse rate patterns for each operator, and uses comparison with said threshold and/or historical measurements for determining if an actual pulsation rate shall belong to a normal situation or to an abnormal situation. Optionally, pulse rate patterns defined for each operator might be used for automatically identifying an operator and activating the locking system of the door, for instance for automatically opening the door when said operator is identified, prohibiting therefore access to the cabin to non-identified operators.

According to the present invention, the triggering of the safety system 4 by the processing unit 33 is preferentially configured for activating an alert for the operator, e.g. using a buzz, a bell, or vibrating or luminous mechanisms, making for instance the portable device 3 vibrating, when at least the following situations occur:
(e) if the processing unit determines notably after a predetermined period of time that there are
   - normal pulsations from the pulsation rate data, and
   - no detectable movement from the first set of data, for instance and in particular, because it does not receive data belonging to said first set of data or because the body part is immobile, and
   - a correct body position from the second set of data, then the triggering of said safety system 4 by the processing unit is configured for activating an alert for the operator (indeed, the possible causes of the above-mentioned situation might be that the operator is not moving the so-called body part wearing the accelerator from a long period or he could be unconscious, but the second set of data did not allow the processing unit to detect this abnormal situation); or
(f) if the processing unit determines notably after a predetermined period of time that there are
   - no or abnormal pulsations from the pulsation rate data, for instance and in particular, if it does not receive any pulsation rate data, and
   - a movement from the first set of data and
   - a correct body position from the second set of data,
   then the triggering of said safety system 4 by the processing unit is configured for activating an alert for the operator (indeed, the possible causes of the above-mentioned situation might be a problem with the pulse rate device); or
(g) if the processing unit determines notably after a predetermined period of time that there are
   - normal pulsations from the pulsation rate data, and
   - a movement from the first set of data, and
   - an incorrect body position from the second set of data, for instance and in particular, if it does not receive data belonging to said second set of data,
   then the triggering of said safety system 4 by the processing unit is configured for activating an alert for the operator (indeed, the possible causes of the above-mentioned situation might be that the operator is not paying attention to the driving).

Preferentially, after another predetermined period of time following a triggering of the safety system that is configured for activating an alert for the operator, the dead man's control system according to the invention is configured for rechecking the pulsation rate data and movement detection data, and if the situation exposed under (e)-(f) did not change, or if a situation falling under the conditions previously exposed under (a)-(d) occurs, then the processing unit is configured for performing another triggering of the safety system 4 wherein said other triggering is configured for activating the emergency brake system 51.

Optionally, the triggering of the safety system 4 by the processing unit 3 is configured for deactivating the locking system of the door 12 of the operator cabin if the processing unit 33 determines that there is no pulsation from the pulsation rate data and/or there is an incorrect body position from the second set of data. Such situations might occur if the operator left the cabin, or if the operator is in an incapacity state regarding the driving of the vehicle. Advantageously, this ensures having access to the cabin in case of critical situations involving an operator.

According to the present invention, the processing unit 33 is configured for correlating the pulsation rate data, the first set of data and the second set of data in order to determine in real time if the operator is capable (normal situation) or not (abnormal situation) of operating the vehicle. Preferentially, learning algorithm are used for improving the speed of the detection of the situation (i.e. of classifying the situation in the class of a "normal situation" or in the class of an "abnormal situation") in order to trigger the right action regarding the safety system 4. In particular, if the processing unit determines that the pulsation of the operator is normal from said pulsation rate data, that a movement of said body part occurs from said first set of data and that the body position of the operator is correct from said second set of data, then the processing unit is configured for not triggering the safety device, since the situation is normal and the operator is conscious and fully capable of driving said vehicle.

Advantageously, the information inputs provided by the pulsation rate data, the first set of data and the second set of data allow the processing unit to safely and accurately determine a state of the operator regarding his driving capacity, i.e. to determine if the situation which occurs is normal or abnormal. Preferentially, wireless communication is used for the transmission of the movement detection data and pulsation rate data. Additionally, the dead man's control system may comprise proximity detection data configured for determining and detecting a presence of the portable device in proximity of the cabin. Additionally, the dead man's control system according to the invention is configured for automatically starting-up the vehicle engine, notably after detecting, by means of the processing unit, a presence of the portable device 3 inside the cabin, and if and only if the processing unit determines normal pulsations from the pulsation rate data and a correct body position from the second set of data. Advantageously, using different sources of data for determining the capacity of the operator to operate the vehicle allows to decrease the time needed for safely evaluating a situation. Additionally, since the drive has no active action to take, he can concentrate on other tasks instead of having for instance to push a confirmation button.

## Claims

1. Dead man's control system configured for checking a capacity of an operator (2) to operate or drive a vehicle (1), the dead man's control system comprising:
- a pulsation rate measurement device (31) configured for measuring heartbeat of the operator (2) and outputting in real time pulsation rate data;
- a movement detection system (32) for detecting a change of position of at least one part of the body of the operator (2) and outputting in real time movement detection data, wherein the movement detection system (32) comprises a camera system comprising at least one camera (321, 322) configured for being installed inside the operator cabin in order to acquire images of the operator, said camera system being configured for enabling a check of the capacity of the operator to drive the vehicle (1), the movement detection data comprising a second set of data that are video data encoding the acquired images provided in real time to the processing unit (33);
- a processing unit (33) for analyzing in real time the pulsation rate data and the movement detection data, wherein the processing unit (33) is configured for automatically triggering a safety system (4) of the vehicle in function of its analysis of the pulsation rate data and the movement detection data wherein the triggering of the safety system (4) by the processing unit (3) is configured for deactivating a locking system of a door (12) of the operator cabin if the processing unit (33) determines that there is no pulsation from the pulsation rate data and/or there is an incorrect body position from the second set of data.

2. Dead man's control system according to claim 1, wherein the processing unit (33) is configured for determining a presence or absence of at least one operator among operators in said operator cabin from its analysis of said pulsation rate data and/or movement detection data and for automatically triggering said safety system (4) in case of an absence of said at least one operator among said operators in the cabin or a driving incapacity of each operator remaining inside the cabin.

3. Dead man's control system according to claim 1 or 2, wherein the triggering of the safety system (4) is configured for carrying out at least one of the following actions:
- activating an emergency brake system (51) of the vehicle;
- activating an alert for the operator;
- putting the vehicle in a fail-safe state, for instance by activating an automatic pilot;
- automatically sending an alarm message to an emergency service;
- starting-up the vehicle engine.

4. Dead man's control system according to one of the claims 1 to 3, wherein the movement detection system (32) comprises an accelerometer (320) for detecting motion of said body part, the movement detection data comprising a first set of data that are accelerometer data provided by said accelerometer (320), wherein said accelerometer (320) and the pulsation rate measurement device (31) are comprised in a portable device (3) configured for being worn on said body part of the operator, said portable device being able to wirelessly communicate with the processing unit (33) in order to provide the pulsation rate data and the first set of data to said processing unit (33).

5. Dead man's control system according to claim 1, wherein the triggering of the safety system (4) by the processing unit (33) is configured for activating the emergency brake system (51) if the following conditions are verified:
(a) if the processing unit determines that there are no or abnormal pulsations from the pulsation rate data and there is no movement from the first set of data and there is an incorrect body position from the second set of data; or
(b) if the processing unit determines that there are no or abnormal pulsations from the pulsation rate data and there is no movement from the first set of data, but there is a correct body position from the second set of data; or
(c) if the processing unit determines that there are no or abnormal pulsations from the pulsation rate data and there is a movement from the first set of data, but there is an incorrect body position from the second set of data; or
(d) if the processing unit determines that there are normal pulsations from the pulsation rate data, but there is no movement from the first set of data and there is an incorrect body position from the second set of data.

6. Dead man's control system according to claim 1 or 5, wherein the triggering of the safety system (4) by the processing unit (33) is configured for activating an alert for the operator if the following conditions are verified:
(e) if the processing unit determines that there are normal pulsations from the pulsation rate data, but there is no movement from the first set of data, and there is a correct body position from the second set of data; or
(f) if the processing unit determines that there is no or abnormal pulsations from the pulsation rate data, but there is a movement from the first set of data and there is a correct body position from the second set of data; or
(g) if the processing unit determines that there are normal pulsations from the pulsation rate data and there is a movement from the first set of data, but there is an incorrect body position from the second set of data.

7. Dead man's control system according to claim 6, wherein the processing unit (33) is configured for performing another triggering of the safety system (4) wherein said other triggering is configured for activating the emergency brake system (51) if the conditions exposed under (e), or (g), or (f), remain unchanged or if the conditions exposed under (a) or (b) or (c) or (d) of claim 5 are met.

8. Dead man's control method capable of detecting in real time a driving incapacity of at least one operator (2) of a vehicle (1) during driving of said vehicle (1) by said operator (2), the method comprising:
- measuring in real time the heartbeat of the operator (2) of said vehicle (1) and outputting in real time pulsation rate data regarding said heartbeat measurement;
- detecting in real time a movement of at least one part (21) of the body of the operator (2) and outputting in real time movement detection data for the movement of said at least one part (21) of the body, wherein images of the operator are acquired by a camera (321,322) installed inside the operator cabin, that belongs to a camera system of a movement detection system (32), wherein the camera system enables to check a capacity of the operator to drive the vehicle (1), the movement detection data comprising a second set of data that are video data encoding the acquired images provided in real time to a processing unit (33) for analyzing in real time the pulsation rate data and the movement detection data;
- analyzing in real time the pulsation rate data and the movement detection data by said processing unit (33) ;
- automatically triggering a safety system (4) of the vehicle in function of the analysis of the pulsation rate data and the movement detection data;
wherein the triggering of the safety system (4) by the processing unit (3) is configured for deactivating a locking system of a door (12) of the operator cabin if the processing unit (33) determines that there is no pulsation from the pulsation rate data and/or there is an incorrect body position from the second set of data.

9. Dead man's control method according to claim 8, comprising a determination of a presence or absence of at least one operator among operators in the operator cabin of the vehicle (1) from the analysis of said pulsation rate data and/or movement detection data and for automatically triggering said safety system (4) in case of an absence of said at least one operator among said operators or a determination of a driving incapacity of each operator remaining inside the cabin.

10. Dead man's control method according to claim 8 or 9, wherein the movement detection data comprise a first set of data provided by an accelerometer configured for measuring a motion of said body part and said second set of data provided by the camera system configured for acquiring images of the operator's body.

11. Dead man's control method according to claim 8 to 10, wherein the triggering of the safety system activates an emergency brake system (51) if the following conditions are verified:
(a) if a processing unit determines that there are no or abnormal pulsations from the pulsation rate data and there is no movement from the first set of data and there is an incorrect body position from the second set of data; or
(b) if the processing unit determines that there are no or abnormal pulsations from the pulsation rate data and there is no movement from the first set of data, but there is a correct body position from the second set of data; or
(c) if the processing unit determines that there are no or abnormal pulsations from the pulsation rate data and there is a movement from the first set of data, but there is an incorrect body position from the second set of data; or
(d) if the processing unit determines that there are normal pulsations from the pulsation rate data, but there is no movement from the first set of data and there is an incorrect body position from the second set of data.

12. Dead man's control method according to claims 8 to 11, wherein the triggering of the safety system (4) by a processing unit (33) activates an alert for the operator if the following conditions are verified:
(e) if the processing unit determines that there are normal pulsations from the pulsation rate data, but there is no movement from the first set of data, and there is a correct body position from the second set of data; or
(f) if the processing unit determines that there are no or abnormal pulsations from the pulsation rate data, but there is a movement from the first set of data and there is a correct body position from the second set of data; or
(g) if the processing unit determines that there are normal pulsations from the pulsation rate data and there is a movement from the first set of data, but there is an incorrect body position from the second set of data.

13. Dead man's control method according to claim 12, comprising performing another triggering of the safety system (4) wherein said other triggering is configured for activating the emergency brake system (51) if the conditions exposed under (e), or (g), or (f), remain unchanged or if the conditions exposed under (a) or (b) or (c) or (d) of claim 11 are met.

## Patentansprüche

1. Totmannsteuersystem, das so konfiguriert ist, dass es eine Fähigkeit eines Fahrers (2) überprüft, ein Fahrzeug (1) zu führen oder zu fahren, wobei das Totmannsteuersystem Folgendes umfasst:
- eine Pulsfrequenzmessvorrichtung (31), die so konfiguriert ist, dass sie den Herzschlag des Fahrers (2) misst und in Echtzeit Pulsfrequenzdaten ausgibt,
- ein Bewegungserkennungssystem (32) zum Erkennen einer Haltungsänderung mindestens eines Körperteils des Fahrers (2) und Ausgeben von Bewegungserkennungsdaten in Echtzeit, wobei das Bewegungserkennungssystem (32) ein Kamerasystem mit mindestens einer Kamera (321, 322) umfasst, die so konfiguriert ist, dass sie in der Fahrerkabine installiert werden und so Bilder vom Fahrer aufnehmen kann, wobei das Kamerasystem so konfiguriert ist, dass es ein Überprüfen der Fähigkeit des Fahrers ermöglicht, das Fahrzeug (1) zu fahren, wobei die Bewegungserkennungsdaten einen zweiten Datensatz umfassen, bei dem es sich um Videodaten handelt, die die aufgenommenen Bilder codieren und in Echtzeit für die Verarbeitungseinheit (33) bereitgestellt werden,
- eine Verarbeitungseinheit (33) zum Analysieren der Pulsfrequenzdaten und der Bewegungserkennungsdaten in Echtzeit, wobei die Verarbeitungseinheit (33) so konfiguriert ist, dass sie in Abhängigkeit von ihrer Analyse der Pulsfrequenzdaten und der Bewegungserkennungsdaten automatisch ein Sicherheitssystem (4) des Fahrzeugs auslöst,
wobei das Auslösen des Sicherheitssystems (4) durch die Verarbeitungseinheit (3) so konfiguriert ist, dass ein Verriegelungssystem für eine Tür (12) der Fahrerkabine deaktiviert wird, wenn die Verarbeitungseinheit (33) anhand der Pulsfrequenzdaten feststellt, dass kein Puls vorhanden ist, und/oder anhand des zweiten Datensatzes feststellt, dass eine unzulässige Körperhaltung vorliegt.

2. Totmannsteuersystem nach Anspruch 1, wobei die Verarbeitungseinheit (33) so konfiguriert ist, dass sie anhand ihrer Analyse der Pulsfrequenzdaten und/oder der Bewegungserkennungsdaten eine Anwesenheit oder Abwesenheit mindestens eines der Fahrer in der Fahrerkabine feststellt und bei einer Abwesenheit des mindestens einen der Fahrer in der Kabine oder einer Fahruntüchtigkeit jedes in der Kabine verbliebenen Fahrers automatisch das Sicherheitssystem (4) auslöst.

3. Totmannsteuersystem nach Anspruch 1 oder 2, wobei das Auslösen des Sicherheitssystems (4) so konfiguriert ist, dass mindestens einer der folgenden Schritte durchgeführt wird:
- Aktivieren eines Notbremssystems (51) des Fahrzeugs,
- Aktivieren einer Warnmeldung für den Fahrer,
- Versetzen des Fahrzeugs in einen Sicherheitszustand, zum Beispiel durch Aktivieren eines Autopiloten,
- automatisches Senden einer Alarmmeldung an einen Bereitschaftsdienst,
- Starten des Fahrzeugmotors.

4. Totmannsteuersystem nach einem der Ansprüche 1 bis 3, wobei das Bewegungserkennungssystem (32) einen Beschleunigungsmesser (320) zum Erkennen einer Bewegung des Körperteils umfasst, wobei die Bewegungserkennungsdaten einen ersten Datensatz umfassen, bei dem es sich um von dem Beschleunigungsmesser (320) bereitgestellte Beschleunigungsmesserdaten handelt, wobei sich der Beschleunigungsmesser (320) und die Pulsfrequenzmessvorrichtung (31) in einer tragbaren Vorrichtung (3) befinden, die so konfiguriert ist, dass sie an dem Körperteil des Fahrers getragen werden kann, wobei die tragbare Vorrichtung in der Lage ist, drahtlos mit der Verarbeitungseinheit (33) zu kommunizieren und so die Pulsfrequenzdaten und den ersten Datensatz für die Verarbeitungseinheit (33) bereitzustellen.

5. Totmannsteuersystem nach Anspruch 1, wobei das Auslösen des Sicherheitssystems (4) durch die Verarbeitungseinheit (33) so konfiguriert ist, dass es das Notbremssystem (51) aktiviert, wenn folgende Bedingungen erfüllt sind:
(a) wenn die Verarbeitungseinheit anhand der Pulsfrequenzdaten feststellt, dass kein oder ein abnormer Puls vorliegt, und anhand des ersten Datensatzes feststellt, dass keine Bewegung erfolgt, und anhand des zweiten Datensatzes feststellt, dass eine unzulässige Körperhaltung vorliegt, oder
(b) wenn die Verarbeitungseinheit anhand der Pulsfrequenzdaten feststellt, dass kein oder ein abnormer Puls vorliegt, und anhand des ersten Datensatzes feststellt, dass keine Bewegung erfolgt, aber anhand des zweiten Datensatzes feststellt, dass eine zulässige Körperhaltung vorliegt, oder
(c) wenn die Verarbeitungseinheit anhand der Pulsfrequenzdaten feststellt, dass kein oder ein abnormer Puls vorliegt, und anhand des ersten Datensatzes feststellt, dass eine Bewegung erfolgt, aber anhand des zweiten Datensatzes feststellt, dass eine unzulässige Körperhaltung vorliegt, oder
(d) wenn die Verarbeitungseinheit anhand der Pulsfrequenzdaten feststellt, dass ein normaler Puls vorliegt, aber anhand des ersten Datensatzes feststellt, dass keine Bewegung erfolgt, und anhand des zweiten Datensatzes feststellt, dass eine unzulässige Körperhaltung vorliegt.

6. Totmannsteuersystem nach Anspruch 1 oder 5, wobei das Auslösen des Sicherheitssystems (4) durch die Verarbeitungseinheit (33) so konfiguriert ist, dass es eine Warnmeldung für den Fahrer aktiviert, wenn folgende Bedingungen erfüllt sind:
(e) wenn die Verarbeitungseinheit anhand der Pulsfrequenzdaten feststellt, dass ein normaler Puls vorliegt, aber anhand des ersten Datensatzes feststellt, dass keine Bewegung erfolgt, und anhand des zweiten Datensatzes feststellt, dass eine zulässige Körperhaltung vorliegt, oder
(f) wenn die Verarbeitungseinheit anhand der Pulsfrequenzdaten feststellt, dass kein oder ein abnormer Puls vorliegt, aber anhand des ersten Datensatzes feststellt, dass eine Bewegung erfolgt, und anhand des zweiten Datensatzes feststellt, dass eine zulässige Körperhaltung vorliegt, oder
(g) wenn die Verarbeitungseinheit anhand der Pulsfrequenzdaten feststellt, dass ein abnormer Puls vorliegt, und anhand des ersten Datensatzes feststellt, dass eine Bewegung erfolgt, aber anhand des zweiten Datensatzes feststellt, dass eine unzulässige Körperhaltung vorliegt.

7. Totmannsteuersystem nach Anspruch 6, wobei die Verarbeitungseinheit (33) so konfiguriert ist, dass sie ein weiteres Auslösen des Sicherheitssystems (4) durchführt, wobei das weitere Auslösen so konfiguriert ist, dass das Notbremssystem (51) aktiviert wird, wenn die unter (e) oder (g) oder (f) genannten Bedingungen bestehen bleiben oder die unter (a) oder (b) oder (c) oder (d) in Anspruch 5 genannten Bedingungen erfüllt sind.

8. Totmannsteuerverfahren, das in der Lage ist, in Echtzeit eine Fahruntüchtigkeit mindestens eines Fahrers (2) eines Fahrzeugs (1) zu erkennen, wenn dieser das Fahrzeug (1) fährt, wobei das Verfahren Folgendes umfasst:
- Messen des Herzschlags des Fahrers (2) des Fahrzeugs (1) in Echtzeit und Ausgeben von Pulsfrequenzdaten zur Herzschlagmessung in Echtzeit,
- Erkennen einer Bewegung mindestens eines Körperteils (21) des Fahrers (2) in Echtzeit und Ausgeben von Bewegungserkennungsdaten für die Bewegung des mindestens einen Körperteils (21) in Echtzeit, wobei von einer in der Fahrerkabine installierten Kamera (321, 322), die zu einem Kamerasystem eines Bewegungserkennungssystems (32) gehört, Bilder vom Fahrer aufgenommen werden, wobei das Kamerasystem ein Überprüfen der Fähigkeit des Fahrers ermöglicht, das Fahrzeug (1) zu fahren, wobei die Bewegungserkennungsdaten einen zweiten Datensatz umfassen, bei dem es sich um Videodaten handelt, die die aufgenommenen Bilder codieren und in Echtzeit für eine Verarbeitungseinheit (33) bereitgestellt werden, damit die Pulsfrequenzdaten und die Bewegungserkennungsdaten in Echtzeit analysiert werden können,
- Analysieren der Pulsfrequenzdaten und der Bewegungserkennungsdaten durch die Verarbeitungseinheit (33) in Echtzeit,
- automatisches Auslösen eines Sicherheitssystems (4) des Fahrzeugs in Abhängigkeit von der Analyse der Pulsfrequenzdaten und der Bewegungserkennungsdaten, wobei das Auslösen des Sicherheitssystems (4) durch die Verarbeitungseinheit (3) so konfiguriert ist, dass ein Verriegelungssystem für eine Tür (12) der Fahrerkabine deaktiviert wird, wenn die Verarbeitungseinheit (33) anhand der Pulsfrequenzdaten feststellt, dass kein Puls vorhanden ist, und/oder anhand des zweiten Datensatzes feststellt, dass eine unzulässige Körperhaltung vorliegt.

9. Totmannsteuerverfahren nach Anspruch 8, das Folgendes umfasst: Feststellen einer Anwesenheit oder Abwesenheit mindestens eines der Fahrer in der Fahrerkabine des Fahrzeugs (1) anhand der Analyse der Pulsfrequenzdaten und/oder der Bewegungserkennungsdaten und automatisches Auslösen des Sicherheitssystems (4) bei einer Abwesenheit des mindestens einen der Fahrer oder bei Feststellen einer Fahruntüchtigkeit jedes in der Kabine verbliebenen Fahrers.

10. Totmannsteuerverfahren nach Anspruch 8 oder 9, wobei die Bewegungserkennungsdaten einen ersten Datensatz umfassen, der von einem Beschleunigungsmesser bereitgestellt wird, der so konfiguriert ist, dass er eine Bewegung des Körperteils misst, und den zweiten Datensatz umfassen, der von dem Kamerasystem bereitgestellt wird, das so konfiguriert ist, dass es Bilder vom Körper des Fahrers aufnimmt.

11. Totmannsteuerverfahren nach Anspruch 8 bis 10, wobei durch Auslösen des Sicherheitssystems ein Notbremssystem (51) aktiviert wird, wenn folgende Bedingungen erfüllt sind:
(a) wenn eine Verarbeitungseinheit anhand der Pulsfrequenzdaten feststellt, dass kein oder ein abnormer Puls vorliegt, und anhand des ersten Datensatzes feststellt, dass keine Bewegung erfolgt, und anhand des zweiten Datensatzes feststellt, dass eine unzulässige Körperhaltung vorliegt, oder
(b) wenn die Verarbeitungseinheit anhand der Pulsfrequenzdaten feststellt, dass kein oder ein abnormer Puls vorliegt, und anhand des ersten Datensatzes feststellt, dass keine Bewegung erfolgt, aber anhand des zweiten Datensatzes feststellt, dass eine zulässige Körperhaltung vorliegt, oder
(c) wenn die Verarbeitungseinheit anhand der Pulsfrequenzdaten feststellt, dass kein oder ein abnormer Puls vorliegt, und anhand des ersten Datensatzes feststellt, dass eine Bewegung erfolgt, aber anhand des zweiten Datensatzes feststellt, dass eine unzulässige Körperhaltung vorliegt, oder
(d) wenn die Verarbeitungseinheit anhand der Pulsfrequenzdaten feststellt, dass ein normaler Puls vorliegt, aber anhand des ersten Datensatzes feststellt, dass keine Bewegung erfolgt, und anhand des zweiten Datensatzes feststellt, dass eine unzulässige Körperhaltung vorliegt.

12. Totmannsteuerverfahren nach Anspruch 8 bis 11, wobei durch Auslösen des Sicherheitssystems (4) durch eine Verarbeitungseinheit (33) eine Warnmeldung für den Fahrer aktiviert wird, wenn folgende Bedingungen erfüllt sind:
(e) wenn die Verarbeitungseinheit anhand der Pulsfrequenzdaten feststellt, dass ein normaler Puls vorliegt, aber anhand des ersten Datensatzes feststellt, dass keine Bewegung erfolgt, und anhand des zweiten Datensatzes feststellt, dass eine zulässige Körperhaltung vorliegt, oder
(f) wenn die Verarbeitungseinheit anhand der Pulsfrequenzdaten feststellt, dass kein oder ein abnormer Puls vorliegt, aber anhand des ersten Datensatzes feststellt, dass eine Bewegung erfolgt, und anhand des zweiten Datensatzes feststellt, dass eine zulässige Körperhaltung vorliegt, oder
(g) wenn die Verarbeitungseinheit anhand der Pulsfrequenzdaten feststellt, dass ein abnormer Puls vorliegt, und anhand des ersten Datensatzes feststellt, dass eine Bewegung erfolgt, aber anhand des zweiten Datensatzes feststellt, dass eine unzulässige Körperhaltung vorliegt.

13. Totmannsteuerverfahren nach Anspruch 12, das Folgendes umfasst: Durchführen eines weiteren Auslösens des Sicherheitssystems (4), wobei das weitere Auslösen so konfiguriert ist, dass das Notbremssystem (51) aktiviert wird, wenn die unter (e) oder (g) oder (f) genannten Bedingungen bestehen bleiben oder die unter (a) oder (b) oder (c) oder (d) in Anspruch 11 genannten Bedingungen erfüllt sind.

## Revendications

1. Système de commande d'homme mort configuré afin de vérifier la capacité d'un opérateur (2) à actionner ou à conduire un véhicule (1), le système de commande d'homme mort comprenant :
- un dispositif de mesure de la fréquence cardiaque (31) configuré afin de mesurer les battements du coeur de l'opérateur (2) et d'émettre des données de fréquence cardiaque en temps réel ;
- un système de détection de mouvement (32) permettant de détecter un changement de position d'au moins une partie du corps de l'opérateur (2) et d'émettre des données de détection de mouvement en temps réel, dans lequel le système de détection de mouvement (32) comprend un système de caméra comprenant au moins une caméra (321, 322) configuré afin d'être installé à l'intérieur de la cabine opérateur afin d'acquérir des images de l'opérateur, ledit système de caméra étant configuré afin de permettre un contrôle de la capacité de l'opérateur à conduire le véhicule (1), les données de détection de mouvement comprenant un second ensemble de données qui sont des données vidéo codant les images acquises fournies en temps réel à l'unité de traitement (33) ;
- une unité de traitement (33) permettant d'analyser en temps réel les données de fréquence cardiaque et les données de détection de mouvement, dans lequel l'unité de traitement (33) est configurée afin d'enclencher automatiquement un système de sécurité (4) du véhicule en fonction de son analyse des données de fréquence cardiaque et des données de détection de mouvement ;
dans lequel le déclenchement du système de sécurité (4) par l'unité de traitement (3) est configuré afin de désactiver un système de verrouillage d'une porte (12) de la cabine opérateur si l'unité de traitement (33) détermine qu'il n'y a pas de pulsation par rapport aux données de fréquence cardiaque et/ou il y a une position du corps incorrecte du second ensemble de données.

2. Système de commande d'homme mort selon la revendication 1, dans lequel l'unité de traitement (33) est configurée afin de déterminer une présence ou l'absence d'au moins un opérateur parmi des opérateurs dans ladite cabine opérateur d'après son analyse desdites données de fréquence cardiaque et/ou de données de détection de mouvement et afin de déclencher automatiquement ledit système de sécurité (4) en cas d'absence dudit au moins un opérateur parmi lesdits opérateurs dans la cabine ou d'une incapacité de conduite de chaque opérateur restant à l'intérieur de la cabine.

3. Système de commande d'homme mort selon la revendication 1 ou 2, dans lequel le déclenchement du système de sécurité (4) est configuré afin de réaliser au moins une des actions suivantes :
- l'activation du système de frein d'urgence (51) du véhicule ;
- l'activation d'une alerte pour l'opérateur ;
- la mise du véhicule dans un état de sécurité intégrée, par exemple en activant un pilote automatique ;
- l'envoi automatique d'un message d'alarme à un service d'urgence ;
- le démarrage du moteur de véhicule.

4. Système de commande d'homme mort selon l'une quelconque des revendications 1 à 3, dans lequel le système de détection de mouvement (32) comprend un accéléromètre (320) permettant de détecter un mouvement de ladite partie de corps, les données de détection de mouvement comprenant un premier ensemble de données qui sont des données d'accéléromètre fournies par ledit accéléromètre (320), dans lequel ledit accéléromètre (320) et le dispositif de mesure de la fréquence cardiaque (31) sont compris dans un dispositif portable (3) configuré afin d'être porté sur ladite partie de corps de l'opérateur, ledit dispositif portable étant capable de communiquer sans fil avec l'unité de traitement (33) afin de fournir les données de fréquence cardiaque et le premier ensemble de données à ladite unité de traitement (33).

5. Système de commande d'homme mort selon la revendication 1, dans lequel le déclenchement du système de sécurité (4) par l'unité de traitement (33) est configuré afin d'activer le système de frein d'urgence (51) si les conditions suivantes sont réunies :
(a) si l'unité de traitement détermine qu'il n'y a pas de pulsations ou qu'il y a des pulsations anormales d'après les données de fréquence cardiaque et qu'il n'y a pas de mouvement d'après le premier ensemble de données et qu'il existe une position incorrecte du corps d'après le second ensemble de données ; ou
(b) si l'unité de traitement détermine qu'il n'y a pas de pulsations ou qu'il y a des pulsations anormales d'après les données de fréquence cardiaque et qu'il n'y a pas de mouvement d'après le premier ensemble de données, mais qu'il existe une position du corps correcte d'après le second ensemble de données ; ou
(c) si l'unité de traitement détermine qu'il n'y pas de pulsations ou qu'il y a des pulsations anormales d'après les données de fréquence cardiaque et qu'il existe un mouvement d'après le premier ensemble de données, mais il existe une position du corps incorrecte d'après le second ensemble de données ; ou
(d) si l'unité de traitement détermine qu'il y a des pulsations normales d'après les données de fréquence cardiaque, mais qu'il n'y a pas de mouvement d'après le premier ensemble de données et qu'il y a une position du corps incorrecte d'après le second ensemble de données.

6. Système de commande d'homme mort selon la revendication 1 ou 5, dans lequel le déclenchement du système de sécurité (4) par l'unité de traitement (33) est configuré afin d'activer une alerte pour l'opérateur si les conditions suivantes se vérifient :
(e) si l'unité de traitement détermine qu'il y a des pulsations normales d'après les données de fréquence cardiaque, mais qu'il n'y a pas de mouvement d'après le premier ensemble de données, et qu'il y a une position correcte du corps par rapport au second ensemble de données ; ou
(f) si l'unité de traitement détermine qu'il n'y a pas de pulsations ou qu'il y a des pulsations anormales d'après les données de fréquence cardiaque, mais qu'il y a un mouvement d'après le premier ensemble de données et qu'il y a une position correcte du corps d'après le second ensemble de données ; ou
(g) si l'unité de traitement détermine qu'il y a des pulsations normales d'après les données de la fréquence cardiaque et qu'il y a un mouvement d'après le premier ensemble de données, mais qu'il y a une position incorrecte du corps d'après le second ensemble de données.

7. Système de commande d'homme mort selon la revendication 6, dans lequel l'unité de traitement (33) est configurée afin de réaliser un autre déclenchement du système de sécurité (4) dans lequel ledit autre déclenchement est configuré afin d'activer le système de frein d'urgence (51) si les conditions exposées aux points (e), ou (g), ou (f), restent inchangées ou si les conditions exposées aux points (a) ou (b) ou (c) ou (d) selon la revendication 5 sont remplies.

8. Procédé de commande d'homme mort capable de détecter en temps réel une incapacité de conduite d'au moins un opérateur (2) d'un véhicule (1) pendant la conduite dudit véhicule (1) par ledit opérateur (2), le procédé comprenant :
- la mesure en temps réel du battement de coeur de l'opérateur (2) dudit véhicule (1) et l'émission en temps réel de données de fréquence cardiaque concernant ladite mesure de battement de coeur ;
- la détection en temps réel d'un mouvement d'au moins une partie (21) du corps de l'opérateur (2) et l'émission en temps réel de données de détection de mouvement pour le mouvement de ladite au moins une partie (21) du corps, dans lequel des images de l'opérateur sont saisies par une caméra (321, 322) installée à l'intérieur de la cabine opérateur, qui appartient à un système de caméra d'un système de détection de mouvement (32), dans lequel le système de caméra permet de vérifier une capacité de l'opérateur à conduire le véhicule (1), les données de détection de mouvement comprenant un second ensemble de données qui sont des données vidéo encodant les images acquises fournies en temps réel à une unité de traitement (33) permettant d'analyser en temps réel les données de fréquence cardiaque et les données de détection de mouvement ;
- l'analyse en temps réel des données de fréquence cardiaque et des données de détection de mouvement par ladite unité de traitement (33) ;
- le déclenchement automatique d'un système de sécurité (4) du véhicule en fonction de l'analyse des données de fréquence cardiaque et des données de détection de mouvement ;
dans lequel le déclenchement du système de sécurité (4) par l'unité de traitement (3) est configuré afin de désactiver un système de verrouillage d'une porte (12) de la cabine opérateur si l'unité de traitement (33) détermine qu'il n'y a pas de pulsation d'après les données de fréquence cardiaque et/ou qu'il y a une position incorrecte du corps d'après le second ensemble de données.

9. Procédé de commande d'homme mort selon la revendication 8, comprenant une détermination d'une présence ou de l'absence d'au moins un opérateur parmi les opérateurs dans la cabine de l'opérateur du véhicule (1) à partir de l'analyse desdites données de fréquence cardiaque et/ou des données de détection de mouvement et pour enclencher automatiquement ledit système de sécurité (4) en cas d'absence dudit au moins un opérateur parmi les opérateurs ou une détermination d'une incapacité de conduite de chaque opérateur restant à l'intérieur de la cabine.

10. Procédé de commande d'homme mort selon la revendication 8 ou 9, dans lequel les données de détection de mouvement comprennent un premier ensemble de données fournies par un accéléromètre configuré afin de mesurer un mouvement de ladite partie du corps et un second ensemble de données fournies par le système de caméra configuré afin de saisir des images du corps de l'opérateur.

11. Procédé de commande d'homme mort selon la revendication 8 à 10, dans lequel le déclenchement du système de sécurité active un système de frein d'urgence (51) si les conditions suivantes sont réunies :
(a) si une unité de traitement détermine qu'il n'y a pas de pulsations ou qu'il y a des pulsations anormales d'après les données de fréquence cardiaque et qu'il n'y a pas de mouvement d'après le premier ensemble de données et qu'il existe une position incorrecte du corps d'après le second ensemble de données ; ou
(b) si l'unité de traitement détermine qu'il n'y a pas de pulsations ou qu'il y a des pulsations anormales d'après les données de fréquence cardiaque et qu'il n'y a pas de mouvement d'après le premier ensemble de données, mais qu'il existe une position du corps correcte d'après le second ensemble de données ; ou
(c) si l'unité de traitement détermine qu'il n'y pas de pulsations ou qu'il y a des pulsations anormales d'après les données de fréquence cardiaque et qu'il existe un mouvement d'après le premier ensemble de données, mais il existe une position du corps incorrecte d'après le second ensemble de données ; ou
(d) si l'unité de traitement détermine qu'il y a des pulsations normales d'après les données de fréquence cardiaque, mais qu'il n'y a pas de mouvement d'après le premier ensemble de données et qu'il y a une position du corps incorrecte d'après le second ensemble de données.

12. Procédé de commande d'homme mort selon les revendications 8 à 11, dans lequel le déclenchement du système de sécurité (4) par une unité de traitement (33) active une alerte pour l'opérateur si les conditions suivantes sont vérifiées :
(e) si l'unité de traitement détermine qu'il y a des pulsations normales d'après les données de fréquence cardiaque, mais qu'il n'y a pas de mouvement d'après le premier ensemble de données, et qu'il y a une position correcte du corps d'après le second ensemble de données ; ou
(f) si l'unité de traitement détermine qu'il n'y a pas de pulsations ou qu'il y a des pulsations anormales d'après les données de fréquence cardiaque, mais qu'il y a un mouvement d'après le premier ensemble de données et qu'il y a une position correcte du corps d'après le second ensemble de données ; ou
(g) si l'unité de traitement détermine qu'il y a des pulsations normales d'après les données de la fréquence cardiaque et il y a un mouvement d'après le premier ensemble de données, mais il y a une position incorrecte du corps d'après le second ensemble de données.

13. Procédé de commande d'homme mort selon la revendication 12, comprenant la réalisation d'un autre déclenchement du système de sécurité (4), dans lequel ledit autre déclenchement est configuré afin d'activer le système de frein d'urgence (51) si les conditions exposées aux points (e), ou (g), ou (f), restent inchangées ou si les conditions exposées aux points (a) ou (b) ou (c) ou (d) selon la revendication 11 sont remplies.
